# EUROPEAN PATENT SPECIFICATION

(11) **EP 3 678 590 B1**
(45) Date of publication and mention of the grant of the patent: **22.01.2025**
(21) Application number: 18792571.4
(22) Date of filing: 06.09.2018
(51) Int. Cl.: A61F 2/08, A61B 17/04, A61B 17/06

(54) **TWO-WAY ADJUSTABLE LOOP SUSPENSORY DEVICE FOR FIXATION OF A SOFT TISSUE GRAFT**
ZWEI-WEGE-VERSTELLBARE SCHLEIFENSUSPENSORIUMSVORRICHTUNG ZUR FIXIERUNG EINES WEICHTEILTRANSPLANTATS
DISPOSITIF SUSPENSEUR À BOUCLE RÉGLABLE BIDIRECTIONNELLE POUR LA FIXATION D'UNE GREFFE DE TISSU MOU

(30) Priority: 07.09.2017 US 201762555081 P
(43) Date of publication of application: 15.07.2020
(73) Proprietor: CONMED Corporation, Largo, FL 33773 (US)
(72) Inventor: KAM, Andrew, Odessa, FL 33556 (US)
(74) Representative: Strehl Schübel-Hopf & Partner
(86) International application number: PCT/US2018/049698
(87) International publication number: WO 2019/051050

(56) References cited:
- EP-A1- 3 323 386
- WO-A2-2012/154922
- FR-A1- 2 980 356
- US-A1- 2010 256 677
- US-A1- 2017 189 007

## Description

### CROSS-REFERENCE TO RELATED APPLICATIONS

This application claims priority to U.S. Provisional Patent Application Serial No. 62/555081, filed on September 7, 2017 and entitled "2-Way Adjustable Loop Suspensory Device."

### BACKGROUND OF THE INVENTION

### 1. Field of the Invention

The present disclosure is directed generally to surgical devices for repair and reconstruction of soft tissue injuries and, more particularly, to devices for fixation of a soft tissue graft at a surgical site.

### 2. Description of Related Art

Many common surgical procedures involve the repair and reconstruction of torn or damaged soft tissue. For example, in common arthroscopic surgical procedures, a replacement graft ligament is secured at the site of the original, now damaged, ligament. The repair and reconstruction of torn or damaged soft tissues is a common surgical procedure. For example, replacement graft ligaments may be secured at the site of the original ligament. The procedure generally involves drilling bone tunnels into adjacent bones at the site of the original ligament and securing a graft ligament within these bone tunnels. In many applications, such as in the knee joint, such procedures may be performed arthroscopically. The graft ligament may be an autograft, an allograft, a xenograft, or it may be totally artificial and synthetic. Common types of anterior cruciate ligament (ACL) grafts, for example, include ones which may be autologous or allograft bone-patellar tendon-bone or soft tissue (such as semitendinosus and gracilis tendons), both types harvested by techniques well known to those skilled in the art.

The graft ligaments may be secured within the bone tunnels in a variety of ways. Of prime importance is the degree to which they can withstand pullout forces prior to complete healing. For example, it is known to use interference screws inserted parallel to the tunnel axis to compress the ends of the graft ligament against the wall of the bone tunnel to secure the graft ligament and promote tissue in-growth.

Suspensory graft fixation devices have been developed to secure a graft ligament in a bone tunnel. One such device is described in U.S. Pat. No. 8,852,250 (Lombardo et al.), entitled Graft Fixation Implant, assigned to the assignee hereof. Suspensory graft fixation devices work by lying transversely across the opening of a bone tunnel and generally take the form of an elongated anchor member which suspends a graft retaining loop from a fixation point on the surface of a bone to which the graft is to be attached (in this case, a femur). The elongated member has an axis and a pair of suture receiving apertures symmetrically situated on the axis on opposite sides of the center of the elongated member. In ACL procedures the elongated member, often called a button, is adapted to be situated transversely across the exit opening of the bone tunnel on the lateral femoral cortex so that a supporting loop, generally made of suture material, can be suspended from the button and can extend into the bone tunnel from the suture receiving apertures of the button. The suture loop supports one end of a graft ligament passed through the loop.

The term "suture" as used herein may be any type of filamentous material such as a biocompatible or bioabsorbable filament, ribbon, tape, woven or non-woven material capable of providing the loop support and the frictional resistance required by the device described herein. In arthroscopic procedures, such as an ACL reconstruction, the elongated anchor member is initially aligned with the axis of the bone tunnel, and pulled through the tunnel to the exit at the distal end on the lateral femur. For such suspensory graft fixation devices to be able to support a graft ligament and to be properly transversely situated at the exit of the bone tunnel, the suture loop and the bone tunnel must both be long enough to enable the elongated member to "flip" from an axially aligned orientation to a transverse orientation when it exits the bone tunnel.

Since the supporting loop of such a suspensory device is most often of a fixed length, graft fixation requires preparation of a graft ligament of predetermined length. Furthermore, because conventional art suspensory graft fixation devices have fixed loop lengths they are produced in multiple sizes (ranging, for example, from loop lengths of 15 mm to 60 mm in 5 mm increments in the case of XO Button^{®} implants made by ConMed Corporation, Largo, Fla.) in order to accommodate various graft and tunnel lengths that may be encountered during a surgical procedure. The fixed graft length and variations in tunnel and loop lengths can make conventional suspensory ligament fixation challenging.

Recently, suspensory devices have been made with adjustable loop lengths. See, for example, U.S. patent application 2010/0256677, (Albertorio et al.) published Oct. 7, 2010 and entitled Integrated Adjustable Button-Suture-Graft Construct with Two Fixation Devices. It has been found that the adjustability of the loop length of a suspensory graft fixation device may be achieved in a manner considerably less complex than that described in the aforementioned publication.

At times surgeons may encounter situations where they cannot produce a bone tunnel of adequate length to receive a ligament graft suitable for suspensory fixation. A predetermined length of graft ligament is required to engage a predetermined portion of the bone tunnel for proper healing. For example, a so-called short tunnel ACL reconstruction may present a relatively small (narrow) femur which does not enable formation of an adequately long bone tunnel which means, in turn, the suspensory anchor member cannot be advanced far enough out of the tunnel to flip yet keep enough contact between the graft and the bone tunnel wall. Use of an adjustable loop in such situations could nevertheless enable the surgeon to proceed with a suspensory-type repair.

In instances in which soft tissue is to be pulled into a bone tunnel, such as ACL reconstruction, it is desired to have an adjustable loop to simplify the procedure and maximize the bone to soft tissue interface.

FR 2 980 356 A1 discloses a replacement ligament fixation device with two free strands and two loops which are locked by respective sheaths on the loop side of an anchor. A similar device is disclosed in WO 2012/154922 A2. A suture provided with a splice and forming an adjustable and self-locking loop is disclosed in US 2017/189007 A1. EP3323386 belongs to the prior art according to Art. 54(3) EPC and discloses a graft fixation device for securing a replacement graft ligament in a bone tunnel comprising an anchor member and a graft supporting loop element formed of a suture threaded through the apertures of the anchor member, the suture forming first and second loops and a splice extending from the bottom surface of the anchor member in the bone tunnel, while the first and second limbs of the suture extend from the top surface of the anchor member.

### SUMMARY OF THE INVENTION

Embodiments of the present invention recognize that there are potential problems and/or disadvantages with conventional suspensory graft fixation devices (as discussed herein and above). Various embodiments of the present invention may be advantageous in that they may solve or reduce one or more of the potential problems and/or disadvantages discussed herein.

The present disclosure is directed to devices for fixation of a soft tissue graft at a surgical site. It is an object of this invention to produce a suspensory graft ligament repair system suitable for short tunnel repairs.

It is another object of this invention to produce a suspensory graft fixation device adapted to lock the size and position of the graft supporting loop after it has been set at a desired length.

It is also an object of this invention to automatically lock the graft supporting loop by pulling it in one direction relative to the anchor member, and to vary the length of the graft supporting loop, to resize it, by pulling it in the opposite direction.

It is yet another object of this invention to correct for instances of over-tensioning of the graft or if the graft has been advanced too far into the bone tunnel.

The present invention is defined in the independent claims. Further advantageous features are set out in the dependent claims. In one aspect, a suspensory graft fixation device for securing a replacement graft ligament in a bone tunnel is provided. The suspensory graft fixation device includes an elongated anchor member with top and bottom surfaces, and adjacent first and second suture receiving apertures extending from the top surface to the bottom surface thereof. A graft supporting loop element is attached to the anchor member and is formed of a suture having first and second limbs. The suture is threaded through the first and second suture receiving apertures such that first and second loops are formed.

The first and second loops extend from the bottom surface, while the first and second limbs extend from the top surface. A splice is formed in the second limb extending from the top surface and the first limb extends through the splice.

According to another aspect, the suspensory graft fixation device includes an elongated anchor member having a top surface and a bottom surface extending between a first end and a second end. A plurality of apertures extend from the top surface to the bottom surface of the elongated anchor member. At least two of the plurality of apertures are adjacent first and second suture receiving apertures. The device also includes a graft supporting loop element attached to the anchor member. The graft supporting loop element is formed of a suture having a first limb and a second limb. The suture is threaded through the first and second suture receiving apertures such that first and second loops are formed in the suture and extend from the bottom surface of the elongated anchor member, while the first and second limbs extend from the top surface. A splice is formed in the second limb of suture extending from the top surface of the elongated anchor member and the first limb extends through the splice. Tensioning the first loop pulls the first limb through the splice, lengthening the first loop, and tensioning the second loop pulls the splice over the first suture receiving aperture.

In yet another aspect, a method of assembling a suspensory graft fixation device for securing a replacement graft ligament in a bone tunnel is provided. The method includes the steps of: (i) providing an elongated anchor member having a top surface and a bottom surface, and adjacent first and second suture receiving apertures extending from the top surface to the bottom surface thereof; (ii) providing a suture having a first limb and a second limb with a central bight portion therebetween; (iii) passing the first limb through the first suture receiving aperture from the top surface to the bottom surface and thereafter, passing the first limb through the second suture receiving aperture from the bottom surface to the top surface, creating a first loop in the first limb; (iv) passing the second limb through the second suture receiving aperture from the top surface to the bottom surface and thereafter, passing the second limb through the first suture receiving aperture from the bottom surface to the top surface, creating a second loop in the second limb; (v) creating a splice in the second limb extending from the top surface from the first suture receiving aperture; and (vi) passing the first limb through the splice in the second limb.

### BRIEF DESCRIPTION OF THE DRAWINGS

One or more aspects of the present invention are particularly pointed out and distinctly claimed as examples in the claims at the conclusion of the specification. The foregoing and other objects, features, and advantages of the invention are apparent from the following description taken in conjunction with the accompanying drawings in which:
FIG. 1 is a perspective view schematic representation of a suspensory fixation device, according to an embodiment;
FIG. 2 is a top perspective view schematic representation of the anchor member of the suspensory fixation device, according to an embodiment;
FIG. 3 is a cross-sectional side view schematic representation of the suspensory fixation device, according to an embodiment;
FIG. 4 is a close-up perspective view schematic representation of a suspensory fixation device, according to an embodiment; and
FIG. 5 is a top view schematic representation of a suspensory fixation device, according to an embodiment;
FIG. 6 is a side view schematic representation of a suspensory fixation device in a bone tunnel, according to an embodiment;
FIG. 7 is a side view schematic representation of a suspensory fixation device in a first configuration attached to a graft and extending from a distal end of a bone tunnel, according to an embodiment; and
FIG. 8 is a side view schematic representation of a suspensory fixation device in a second configuration attached to a graft and extending from a distal end of a bone tunnel, according to an embodiment.

### DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation.

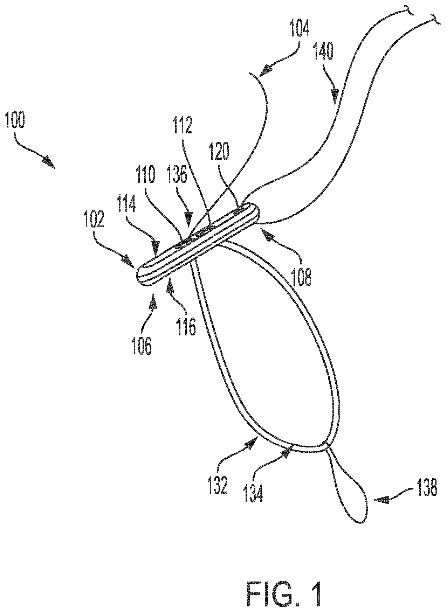

Referring now to the figures, wherein like reference numerals refer to like parts throughout, FIG. 1 shows a side perspective view schematic representation of a suspensory graft fixation device 100, according to an embodiment. The device 100 comprises an elongated anchor member 102 and a length of suture 104. In the depicted embodiment, the suture 104 is in the form of a filamentous strand composed of high strength, filamentous material such as ultrahigh molecular weight polyethylene. The anchor member 102 can be composed of metal, such as implantable grade titanium, or any other suitable bioabsorbable or biocompatible material (as should be understood by a person of ordinary skill in the art in conjunction with a review of this disclosure). In embodiments, the length of anchor member 102 may range from 12 mm to 20 mm.

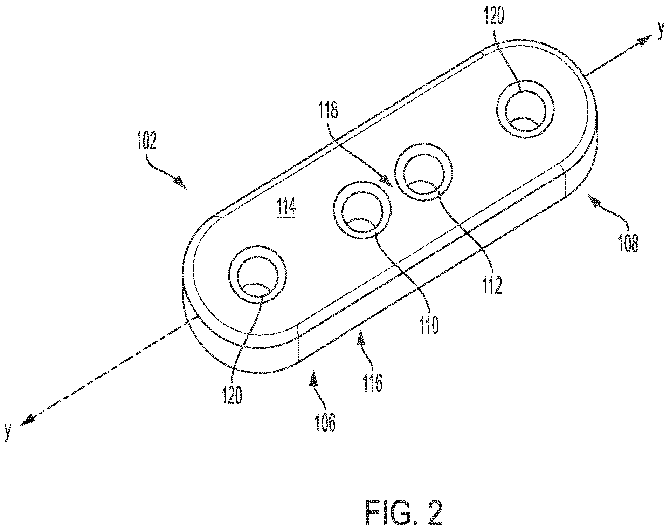

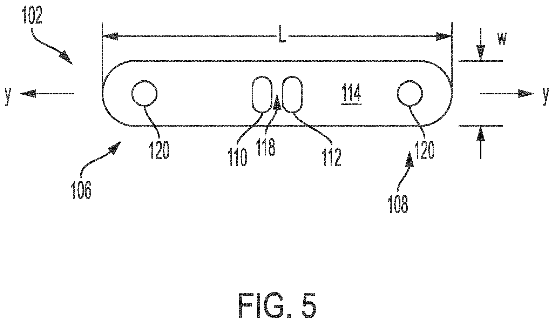

Turning briefly to FIG. 2, there is a top perspective view schematic representation of the anchor member 102 of the suspensory fixation device 100, according to an embodiment. The anchor member 102 extends along a central longitudinal y - y axis between its first end 106 and second end 108. The anchor member 102 also has a pair of central suture receiving apertures 110, 112, which are sized or otherwise configured to receive suture 104 that will form loops. For example, the diameters of suture receiving apertures 110, 112 may be on the order of 1 mm, while the diameter of the suture 104 may be on the order of 1 mm or USP size #5. In an embodiment according to FIG. 5, the anchor member 102 is oblong in geometry. In particular, as shown, a length *L* of the anchor member 102 is greater than a width w of the anchor member 102. The oblong geometry of the anchor member 102 allows the anchor member 102 to pass through narrow bone tunnels.

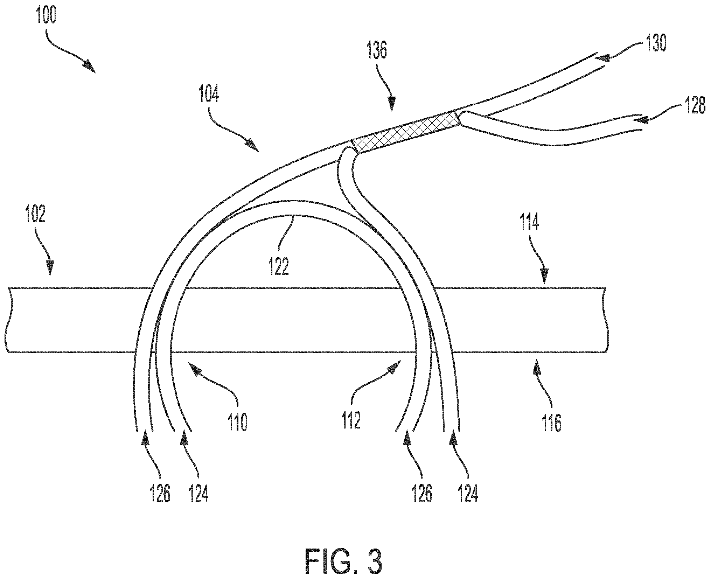

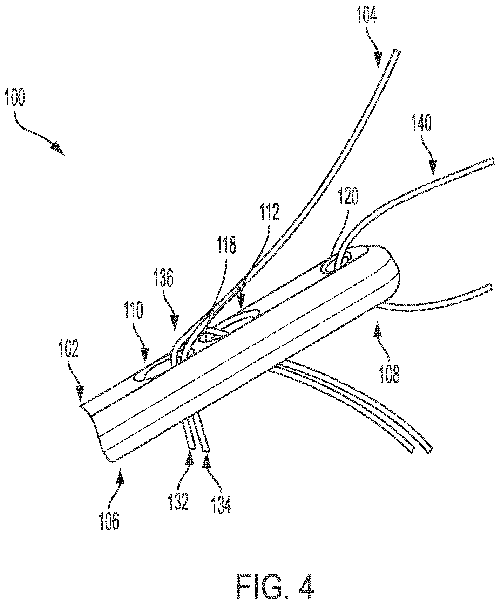

Referring back to FIG. 2, the anchor member 102 has a top surface 114 and a bottom surface 116 (best seen in FIGs. 3-4). The bottom surface 116 is sometimes referred to as the proximal surface and is intended to be placed adjacent a bone tunnel exit. As used herein, the term "proximal" refers to the side of the bone containing the bone tunnel (i.e., extending inwardly away from the surface of the lateral femur in an ACL procedure), and the term "distal" refers to the side of the bone against which the transverse anchor member 102 rests (i.e., extending outwardly away from the surface on the lateral femur).

Still referring to FIG. 2, the suture receiving apertures 110, 112 are situated on opposite sides of a central bridge portion 118 extending between them. The anchor member 102 may also optionally have one or more placement apertures 120 extending between the top and bottom surfaces 114, 116. In the depicted embodiment, there is a placement aperture 120 at the first end 106 of the anchor member 102 and a placement aperture 120 at the second end 108 of the anchor member 102. The placement apertures 120 are sized or otherwise configured to receive a placement suture 140 (or another filamentous strand) to facilitate placement of the device 100 at a bone tunnel exit. For example, a placement suture 140 is attached to a placement aperture 120 and pulled through the bone tunnel, facilitating orienting the elongated anchor member 102 parallel to the bone tunnel axis.

As shown in FIG. 1, the suspensory fixation device 100 is designed to have the anchor member 102 operate with a filamentous strand 104 suitable for following a tortuous path through the suture receiving apertures 110, 112 of anchor member 102. In an embodiment, the filamentous strand 104 is a single length of appropriately sized suture. The term "suture" as used herein may be used interchangeably with "filamentous material" and, as described above, will be understood to mean any biocompatible or bioabsorbable strand of material which can, when combined with anchor member 102, operate to support a replacement graft in the manner described below. As will be understood below, the combination of filamentous strand 104 with the features of anchor member 102 can perform different functions along the path of the suture 104 through the suture receiving apertures 110, 112 of the anchor member 102.

Referring now to FIG. 3, there is a cross-sectional side view schematic representation of the suspensory fixation device 100, according to an embodiment. To load the anchor member 102 shown in FIG. 2, the filamentous strand 104 is passed or wrapped through the suture receiving apertures 110, 112. Specifically, as shown in FIG. 3, the filamentous strand 104 is first folded on itself to form a central bight portion 122, thus creating two limbs 124, 126 extending from the central bight portion 122. Each limb 124, 126 has a length extending from the central bight portion 122 to the free, unattached ends 128, 130 of the limbs 124, 126.

Still referring to FIG. 3, the first limb 124 is passed through a first suture receiving aperture 110 and the second limb 126 is passed through a second suture receiving aperture 112 (in the downward direction as shown in FIG. 3). The first and second limbs 124, 126 extend through the suture receiving apertures 110, 112 from the top surface 114 of the anchor member 102 to the bottom surface 116 of the anchor member 102. The second limb 126 is then passed up through the first suture receiving aperture 110 from the bottom surface 116 of the anchor member 102 to the top surface 114 of the anchor member 102. Similarly, the first limb 124 is passed up through the second suture receiving aperture 112 from the bottom surface 116 of the anchor member 102 to the top surface 114 of the anchor member 102. As shown in FIG. 3, the central bight portion 122 extends over the central bridge portion 118 on the top surface 114 of the anchor member 102, while two adjustable loops 132, 134 extend from the suture receiving apertures 110, 112 through the bottom surface 116 of the anchor member 102, as shown in FIG. 1.

With the free, unattached ends 128, 130 extending from the top surface 114 of the anchor member 102, a splice 136 is created in the second limb 126, as shown in FIG. 3. The first limb 124 is passed through the splice 136, forming a jacket around the first limb 124. In the depicted embodiment, the splice 136 is adjacent and above (distal to) the central bight portion 122. With the filamentous strand 104 wrapped through the suture receiving apertures 110, 112, creating adjustable loops 132, 134 extending from the bottom surface 116 of the anchor member 102, and the splice 136 extending from the top surface 114 of the anchor member 102, the device 100 can be used to adjustably apply and release tension on a graft at a bone tunnel exit.

While the suture path of an embodiment of device 100 is as shown in FIG. 3, alternate embodiments are feasible. Thus, while the suture path through the anchor member 102 results in device 100 comprising a graft supporting element in the form of two adjustable loops 132, 134, there can be different loop constructions than that described above. For example, the adjustable loops 132, 134 are formed from a single length of suture 104 (or other filamentous material) but in an alternate embodiment, the adjustable loops 132, 134 could be formed by a plurality of individual lengths of suture 104 which together form the adjustable loops 132, 134.

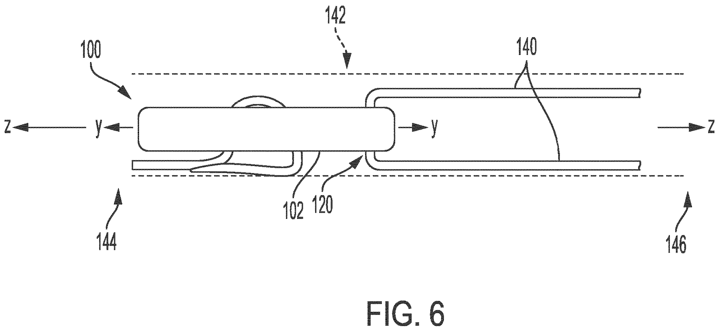

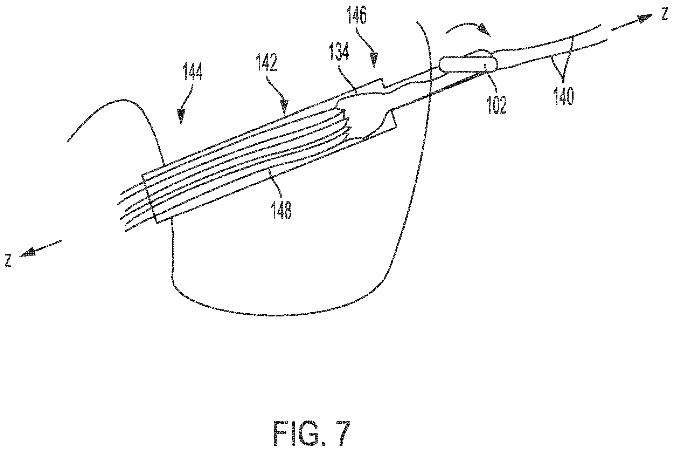

Turning now to FIGs. 6-8, there are shown side views schematic representations of the device 100 at various configurations during deployment. First, the device 100 shown in FIG. 1 is attached to a graft 148 (FIGs. 7-8). In an embodiment, the graft 148 (FIGs. 7-8) is attached to the second loop 134 and a placement suture 140 is threaded through a placement aperture 120 of the anchor member 102. The placement suture 140 is inserted through the proximal end 144 of the bone tunnel 142 and pulled toward the distal end 146 of the bone tunnel 142. FIG. 6 shows a side view schematic representation of the device 100 in a bone tunnel 142, according to an embodiment. As shown, the placement suture 140 is pulled or otherwise tensioned toward the distal end 146 of the bone tunnel 142. In the depicted embodiment, the placement suture 140 is pulled through the bone tunnel 142, facilitating orienting the elongated anchor member 102 substantially parallel to the bone tunnel axis z - z.

Referring now to FIG. 7, there is shown a side view schematic representation of the device 100 in a first configuration attached to a graft 148 and extending from the distal end 146 of the bone tunnel 142. As shown in FIG. 7, the placement suture 140 is pulled until the anchor member 102 exits the distal end 146 of the bone tunnel 142 and the graft 148 remains within the bone tunnel 142. When using the device 100 to secure the graft 148, the two adjustable loops 132, 134 (also shown in FIG. 1) serve different purposes. The first loop 132, which is created from the first limb 124, functions to resize the lengths of both adjustable loops 132, 134. The second loop 134, which is created from the second limb 126 and attached to the graft 148, functions to lock the device 100 in place, thereby locking the graft 148 in position with respect to the bone tunnel exit (i.e., distal end 146 of the bone tunnel 142).

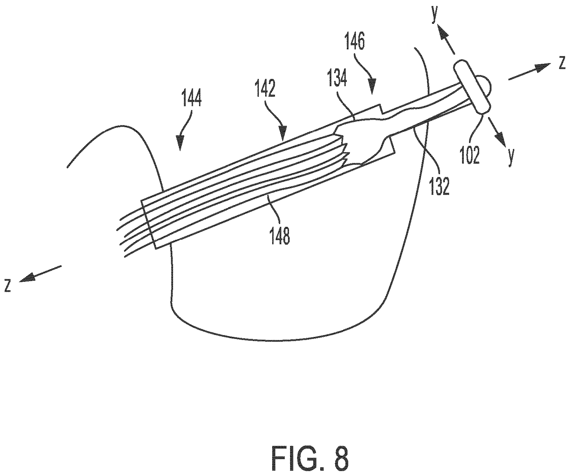

In use, tension is first applied to the first loop 132, which causes an increase in size of both adjustable loops 132, 134. In one embodiment, a tether 138 (e.g., rope, suture, or other filamentous strand) is attached to the first loop 132, as shown in FIG. 1. When the tether 138 is pulled proximally or otherwise away from the splice 136, slack is introduced into the adjustable loops 132, 134, enlarging the adjustable loops 132, 134. In some cases, the splice 136 is pulled toward the second suture receiving aperture 112 when the first loop 132 is tensioned. However, the splice 136 is too large relative to the second suture receiving aperture 112 and can thus not be pulled through the second suture receiving aperture 112 to the bottom surface 116 of the anchor member 102. Accordingly, additional tension on the first loop 132, pulls the first limb 124 (and the splice 136) proximally to the second suture receiving aperture 112 where the first limb 124 is, due to its size relative to the second suture receiving aperture 112, pulled through the splice 136 and the second suture receiving aperture 112 to provide the extra slack in the adjustable loops 132, 134. At the same time, tension on both adjustable loops 132, 134, by the graft 148, causes the anchor member 102 to rotate. As shown in FIGs. 7-8, the graft 148 (or filament (not shown) attached to the graft 148) can be tensioned or otherwise pulled proximally from the proximal end 144 of the bone tunnel 142, which tensions the adjustable loops 132, 134. The tension from the graft 148 the anchor member 102 to rotate from a first configuration substantially parallel to the bone tunnel axis z - z (FIG. 7) to a second configuration substantially perpendicular to the bone tunnel axis z - z (FIG. 8). When the anchor member 102 is perpendicular across the bone tunnel 142, the anchor member 102 is locked in place by bringing the splice 136 against the first suture receiving aperture 110. Again, the splice 136 is too large relative to the first suture receiving aperture 110 and can thus not be pulled through the first suture receiving aperture 110 to the bottom surface 116 of the anchor member 102. As the splice 136 is created in the second limb 126, the second limb 126 cannot be pulled through the first suture receiving aperture 110 to supply slack and instead locks the device 100.

While various embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific embodiments described herein. It is, therefore, to be understood that the foregoing embodiments are presented by way of example only and that, within the scope of the appended claims embodiments may be practiced otherwise than as specifically described and claimed. Embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as, "has" and "having"), "include" (and any form of include, such as "includes" and "including"), and "contain" (any form of contain, such as "contains" and "containing") are open-ended linking verbs. As a result, a method or device that "comprises", "has", "includes" or "contains" one or more steps or elements. Likewise, a step of method or an element of a device that "comprises", "has", "includes" or "contains" one or more features possesses those one or more features, but is not limited to possessing only those one or more features.

The description of the present invention has been presented for purposes of illustration and description, but is not intended to be exhaustive or limited to the invention in the form disclosed. Many modifications and variations will be apparent to those of ordinary skill in the art without departing from the scope of the invention, as defined by the appended claims. The embodiment was chosen and described in order to best explain the principles of one or more aspects of the invention and the practical application, and to enable others of ordinary skill in the art to understand one or more aspects of the present invention for various embodiments with various modifications as are suited to the particular use contemplated.

## Claims

1. A suspensory graft fixation device for securing a replacement graft ligament in a bone tunnel, comprising:
an elongated anchor member (102) comprising a top surface (114) and a bottom surface (116), and adjacent first and second suture receiving apertures (110, 112) extending from the top surface to the bottom surface thereof;
a graft supporting loop element attached to the anchor member, the graft supporting loop element formed of a suture (104) comprising a first limb (124) and a second limb (126);
wherein the suture is threaded through the first and second suture receiving apertures forming first and second loops (132, 134) extending from the bottom surface of the elongated anchor member, while the first and second limbs extend from the top surface,
**characterized in that** it comprises a splice (136) formed in the second limb of suture extending from the top surface of the elongated anchor member, wherein the first limb extends through the splice and wherein the splice is too large relative to the first and second suture receiving apertures and cannot be pulled through to the bottom surface of the elongated anchor member.

2. The suspensory graft fixation device of claim 1, further comprising a central bight portion (122) extending between the first limb (124) and the second limb (126), the central bight portion extending over a central bridge portion (118) of the elongated anchor member (102) between the first and second suture receiving apertures (110, 112).

3. The suspensory graft fixation device of claim 1, wherein the first limb (124) extends from the top surface (114) to the bottom surface (116) through the first suture receiving aperture (110) and from the bottom surface to the top surface through the second suture receiving aperture (112).

4. The suspensory graft fixation device of claim 1, wherein the second limb (126) extends from the top surface (114) to the bottom surface (116) through the second suture receiving aperture (112) and from the bottom surface to the top surface through the first suture receiving aperture (110).

5. The suspensory graft fixation device of claim 1, wherein the first loop (132) is formed by the first limb (124).

6. The suspensory graft fixation device of claim 1, wherein the second loop (134) is formed by the second limb (126).

7. The suspensory graft fixation device of claim 1, wherein the elongated anchor member (102) is composed of titanium.

8. The suspensory graft fixation device of any one of the preceding claims, wherein:
the top surface (114) and the bottom surface (116) of the elongated anchor member (102) extend between a first end and a second end of the elongated anchor member;
wherein the first loop (132) is formed so that tensioning the first loop pulls the first limb (124) through the splice (136), lengthening the first loop; and
wherein the second loop (134) is formed so that tensioning the second loop pulls the splice over the first suture receiving aperture (110).

9. The suspensory graft fixation device of claim 8, further comprising a placement aperture (120) at either the first end or the second end of the elongated anchor member (102) extending from the top surface (114) to the bottom surface (116), wherein the placement aperture is configured to receive a placement suture (140) therethrough.

10. The suspensory graft fixation device of claim 8, further comprising a tether (138) connected to the first loop (132).

11. A method of assembling a suspensory graft fixation device for securing a replacement graft ligament in a bone tunnel, comprising the steps of:
providing an elongated anchor member (102) having a top surface (114) and a bottom surface (116), and adjacent first and second suture receiving apertures (110, 112) extending from the top surface to the bottom surface thereof;
providing a suture (104) having a first limb (124) and a second limb (126) with a central bight portion (122) therebetween;
passing the first limb through the first suture receiving aperture from the top surface to the bottom surface and thereafter, passing the first limb through the second suture receiving aperture from the bottom surface to the top surface, creating a first loop (132) in the first limb;
passing the second limb through the second suture receiving aperture from the top surface to the bottom surface and thereafter, passing the second limb through the first suture receiving aperture from the bottom surface to the top surface, creating a second loop (134) in the second limb;
creating a splice (136) in the second limb extending from the top surface from the first suture receiving aperture; and
passing the first limb through the splice in the second limb;
wherein the splice is larger than the first and second suture receiving apertures of the elongated anchor member and cannot be pulled through to the bottom surface of the elongated anchor member.

12. The method of claim 11, further comprising the step of tensioning the first loop (132), thereby enlarging the first loop.

13. The method of claim 12, wherein the step of tensioning the first loop (132), thereby enlarging the first loop includes the step of pulling the first limb (124) through splice (136) toward the bottom surface (116).

14. The method of claim 12, further comprising the step of tensioning the second loop (134) after tensioning the first loop (124), which pulls the splice (136) to the first suture receiving aperture (110).

15. The method of claim 12, wherein the step of tensioning the first loop (132), thereby enlarging the first loop includes the step of pulling a tether (138) connected to the first loop.

## Patentansprüche

1. Vorrichtung zur suspensiven Transplantatfixierung zum Befestigen eines Ersatztransplantatbands in einem Knochentunnel, umfassend:
ein längliches Verankerungselement (102), das eine obere Fläche (114) und eine untere Fläche (116) sowie benachbarte erste und eine zweite Fadenaufnahmeöffnungen (110, 112), die sich von der oberen Fläche zur unteren Fläche erstrecken, umfasst;
ein am Verankerungselement angebrachtes Transplantat-Stützschlaufenelement, wobei das Transplantat-Stützschlaufenelement aus einem Faden (104) gebildet ist, der einen ersten Schenkel (124) und einen zweiten Schenkel (126) umfasst;
wobei der Faden durch die ersten und zweiten Fadenaufnahmeöffnungen gefädelt wird, die erste und zweite Schlaufen (132, 134) bilden, die von der unteren Fläche des länglichen Verankerungselements aus verlaufen, während der erste und der zweite Schenkel von der oberen Fläche aus verlaufen,
**gekennzeichnet dadurch, dass** sie eine Spleißung (136) umfasst, die im zweiten Schenkel des Fadens, der von der oberen Fläche des länglichen Verankerungselements verläuft, gebildet ist, wobei der erste Schenkel durch die Spleißung hindurch verläuft und wobei die Spleißung im Verhältnis zu den ersten und zweiten Fadenaufnahmeöffnungen zu groß ist und nicht zur unteren Fläche des länglichen Verankerungselements durchgezogen werden kann.

2. Vorrichtung zur suspensiven Transplantatfixierung nach Anspruch 1, ferner umfassend einen zentralen Biegeabschnitts (122), der zwischen dem ersten Schenkel (124) und dem zweiten Schenkel (126) verläuft, wobei der zentrale Biegeabschnitt über einen zentralen Brückenabschnitt (118) des länglichen Verankerungselements (102) zwischen den ersten und zweiten Fadenaufnahmeöffnungen (110, 112) verläuft.

3. Vorrichtung zur suspensiven Transplantatfixierung nach Anspruch 1, wobei der erste Schenkel (124) von der oberen Fläche (114) zur unteren Fläche (116) durch die erste Fadenaufnahmeöffnung (110) und von der unteren Fläche zur oberen Fläche durch die zweite Fadenaufnahmeöffnung (112) verläuft.

4. Vorrichtung zur suspensiven Transplantatfixierung nach Anspruch 1, wobei der zweite Schenkel (126) von der oberen Fläche (114) zur unteren Fläche (116) durch die zweite Fadenaufnahmeöffnung (112) und von der unteren Fläche zur oberen Fläche durch die erste Fadenaufnahmeöffnung (110) verläuft.

5. Vorrichtung zur suspensiven Transplantatfixierung nach Anspruch 1, wobei die erste Schlaufe (132) vom ersten Schenkel (124) gebildet wird.

6. Vorrichtung zur Fixierung eines Hüfttransplantats nach Anspruch 1, wobei die zweite Schlaufe (134) vom zweiten Schenkel (126) gebildet wird.

7. Vorrichtung zur suspensiven Transplantatfixierung nach Anspruch 1, wobei das längliche Verankerungselement (102) aus Titan besteht.

8. Vorrichtung zur suspensiven Transplantatfixierung nach einem der vorhergehenden Ansprüche, wobei:
die obere Fläche (114) und die untere Fläche (116) des länglichen Verankerungselements (102) zwischen einem ersten Ende und einem zweiten Ende des länglichen Verankerungselements verlaufen;
wobei die erste Schlaufe (132) so gebildet ist, dass das Spannen der ersten Schlaufe den ersten Schenkel (124) durch die Spleißung (136) zieht, wodurch die erste Schlaufe verlängert wird; und
wobei die zweite Schlaufe (134) so gebildet ist, dass das Spannen der zweiten Schlaufe die Spleißung über die erste Fadenaufnahmeöffnung (110) zieht.

9. Vorrichtung zur suspensiven Transplantatfixierung nach Anspruch 8, ferner umfassend eine Platzierungsöffnung (120) entweder am ersten Ende oder am zweiten Ende des länglichen Verankerungselements (102), die von der oberen Fläche (114) zur unteren Fläche (116) verläuft, wobei die Platzierungsöffnung so konfiguriert ist, dass sie einen Platzierungsfaden (140) durch sie hindurch aufnehmen kann.

10. Vorrichtung zur suspensiven Transplantatfixierung nach Anspruch 8, ferner umfassend eine mit der ersten Schlaufe (132) verbundene Leine (138).

11. Verfahren zur Zusammensetzung einer Vorrichtung zur suspensiven Transplantatfixierung zum Befestigen eines Ersatztransplantatbands in einem Knochentunnel, umfassend die folgenden Schritte:
Bereitstellen eines länglichen Verankerungselements (102) mit einer oberen Fläche (114) und einer unteren Fläche (116) und benachbarten ersten und zweiten Fadenaufnahmeöffnungen (110, 112), die von der oberen Fläche zur unteren Fläche verlaufen.
Bereitstellen eines Fadens (104) mit einem ersten Schenkel (124) und einem zweiten Schenkel (126) mit einem dazwischen liegenden zentralen Biegeabschnitt (122);
Durchführen des ersten Schenkels durch die erste Fadenaufnahmeöffnung von der oberen Fläche zur unteren Fläche und danach Durchführen des ersten Schenkels durch die zweite Fadenaufnahmeöffnung von der unteren Fläche zur oberen Fläche, wodurch eine erste Schlaufe (132) im ersten Schenkel entsteht;
Durchführen des zweiten Schenkels durch die zweite Fadenaufnahmeöffnung von der oberen Fläche zur unteren Fläche und danach Durchführen des zweiten Schenkels durch die erste Fadenaufnahmeöffnung von der unteren Fläche zur oberen Fläche, wodurch eine zweite Schlaufe (134) im zweiten Schenkel entsteht;
Erzeugen einer Spleißung (136) im zweiten Schenkel, die von der oberen Fläche von der ersten Fadenaufnahmeöffnung aus verläuft; und
Durchführen des ersten Schenkels durch die Spleißung im zweiten Schenkel;
wobei die Spleißung größer ist als die ersten und zweiten Fadenaufnahmeöffnungen des länglichen Verankerungselements ist und nicht zur unteren Fläche des länglichen Verankerungselements durchgezogen werden kann.

12. Verfahren nach Anspruch 11 umfasst ferner den Schritt des Spannens der ersten Schlaufe (132), wodurch die erste Schlaufe vergrößert wird.

13. Verfahren nach Anspruch 12, wobei der Schritt des Spannens der ersten Schlaufe (132), wodurch die erste Schlaufe vergrößert wird, den Schritt des Ziehens des ersten Schenkels (124) durch die Spleißung (136) in Richtung der unteren Fläche (116) umfasst.

14. Verfahren nach Anspruch 12, ferner umfassend den Schritt des Spannens der zweiten Schlaufe (134) nach dem Spannen der ersten Schlaufe (124), wodurch die Spleißung (136) zur ersten Fadenaufnahmeöffnung (110) gezogen wird.

15. Verfahren nach Anspruch 12, wobei der Schritt des Spannens der ersten Schlaufe (132), wodurch die erste Schlaufe vergrößert wird, den Schritt des Ziehens an einer mit der ersten Schlaufe verbundenen Leine (138) umfasst.

## Revendications

1. Dispositif suspenseur pour fixation de greffon pour immobiliser un greffon ligamentaire de remplacement dans un canal d'os, comprenant :
un élément d'ancrage allongé (102) comprenant une surface supérieure (114) et une surface de fond (116), et des première et deuxième ouvertures adjacentes (110, 112) de réception de suture s'étendant depuis la surface supérieure jusqu'à la surface de fond de celui-ci ;
un élément en boucle de support de greffon fixé à l'élément d'ancrage, l'élément en boucle de support de greffon constitué d'une suture (104) comprenant un premier brin (124) et un deuxième brin (126) ;
dans lequel la suture est passée par filetage à travers les première et deuxième ouvertures de réception de suture formant des première et deuxième boucles (132, 134) s'étendant depuis la surface de fond de l'élément d'ancrage allongé, tandis que les premier et deuxième brins s'étendent depuis la surface supérieure,
**caractérisé en ce qu'**il comprend une épissure (136) formée dans le deuxième brin de suture s'étendant depuis la surface supérieure de l'élément d'ancrage allongé, dans lequel le premier brin s'étend à travers l'épissure et dans lequel l'épissure est trop grande par rapport aux première et deuxième ouvertures de réception de suture et ne peut être tirée à travers la surface de fond de l'élément d'ancrage allongé.

2. Dispositif suspenseur pour fixation de greffon selon la revendication 1, comprenant en outre une partie centrale de boucle (122) s'étendant entre le premier brin (124) et le deuxième brin (126), la partie centrale de boucle s'étendant par-dessus une partie centrale de pont (118) de l'élément d'ancrage allongé (102) entre les première et deuxième ouvertures (110, 112) de réception de suture.

3. Dispositif suspenseur pour fixation de greffon selon la revendication 1, dans lequel le premier brin (124) s'étend depuis la surface supérieure (114) jusqu'à la surface de fond (116) à travers la première ouverture (110) de réception de suture et depuis la surface de fond jusqu'à la surface supérieure à travers la deuxième ouverture (112) de réception de suture.

4. Dispositif suspenseur pour fixation de greffon selon la revendication 1, dans lequel le deuxième brin (126) s'étend depuis la surface supérieure (114) jusqu'à la surface de fond (116) à travers la deuxième ouverture (112) de réception de suture et depuis la surface de fond jusqu'à la surface supérieure à travers la première ouverture (110) de réception de suture.

5. Dispositif suspenseur pour fixation de greffon selon la revendication 1, dans lequel la première boucle (132) est formée par le premier brin (124).

6. Dispositif suspenseur pour fixation de greffon selon la revendication 1, dans lequel la deuxième boucle (134) est formée par le deuxième brin (126).

7. Dispositif suspenseur pour fixation de greffon selon la revendication 1, dans lequel l'élément d'ancrage allongé (102) est composé de titane.

8. Dispositif suspenseur pour fixation de greffon selon l'une quelconque des revendications précédentes, dans lequel :
la surface supérieure (114) et la surface de fond (116) de l'élément d'ancrage allongé (102) s'étendent entre une première extrémité et une deuxième extrémité de l'élément d'ancrage allongé ;
dans lequel la première boucle (132) est formée de telle manière que la mise en tension de la première boucle tire le premier brin (124) à travers l'épissure (136), allongeant la première boucle ; et
dans lequel la deuxième boucle (134) est formée de telle manière que la mise en tension de la deuxième boucle tire l'épissure par-dessus la première ouverture (110) de réception de suture.

9. Dispositif suspenseur pour fixation de greffon selon la revendication 8, comprenant en outre une ouverture (120) de mise en place à l'une ou l'autre de la première extrémité ou de la deuxième extrémité de l'élément d'ancrage allongé (102) s'étendant depuis la surface supérieure (114) jusqu'à la surface de fond (116), dans lequel l'ouverture de mise en place est configurée pour recevoir une suture (140) de mise en place à travers celle-ci.

10. Dispositif suspenseur pour fixation de greffon selon la revendication 8, comprenant en outre une sangle (138) connectée à la première boucle (132).

11. Procédé d'assemblage d'un dispositif suspenseur pour fixation de greffon pour immobiliser un greffon ligamentaire de remplacement dans un canal d'os, comprenant les étapes de :
prévision d'un élément d'ancrage allongé (102) ayant une surface supérieure (114) et une surface de fond (116), et de première et deuxième ouvertures adjacentes (110, 112) de réception de suture s'étendant depuis la surface supérieure jusqu'à la surface de fond de celui-ci ;
prévision d'une suture (104) ayant un premier brin (124) et un deuxième brin (126) avec une partie centrale de boucle (122) entre ceux-ci ;
passage du premier brin à travers la première ouverture de réception de suture depuis la surface supérieure jusqu'à la surface de fond et ensuite, passage du premier brin à travers la deuxième ouverture de réception de suture depuis la surface de fond jusqu'à la surface supérieure, créant une première boucle (132) dans le premier brin ;
passage du deuxième brin à travers la deuxième ouverture de réception de suture depuis la surface supérieure jusqu'à la surface de fond et ensuite, passage du deuxième brin à travers la première ouverture de réception de suture depuis la surface de fond jusqu'à la surface supérieure, créant une deuxième boucle (134) dans le deuxième brin ;
création d'une épissure (136) dans le deuxième brin s'étendant depuis la surface supérieure depuis la première ouverture de réception de suture ; et
passage du premier brin à travers l'épissure dans le deuxième brin ;
dans lequel l'épissure est plus grande que les première et deuxième ouvertures de réception de suture de l'élément d'ancrage allongé et ne peut être tirée jusqu'à la surface de fond de l'élément d'ancrage allongé.

12. Procédé selon la revendication 11, comprenant en outre l'étape de mise en tension de la première boucle (132), agrandissant ainsi la première boucle.

13. Procédé selon la revendication 12, dans lequel l'étape de mise en tension de la première boucle (132), agrandissant ainsi la première boucle, inclut l'étape de traction du premier brin (124) à travers l'épissure (136) vers la surface de fond (116).

14. Procédé selon la revendication 12, comprenant en outre l'étape de mise en tension de la deuxième boucle (134) après mise en tension de la première boucle (132), qui tire l'épissure (136) jusqu'à la première ouverture (110) de réception de suture.

15. Procédé selon la revendication 12, dans lequel l'étape de mise en tension de la première boucle (132), agrandissant ainsi la première boucle, inclut l'étape de traction d'une sangle (138) connectée à la première boucle.
